# FASCICULE DE BREVET EUROPEEN

(11) **EP 1 040 791 B1**
(45) Date de publication et mention de la délivrance du brevet: **31.05.2006**
(21) Numéro de dépôt: 00400843.9
(22) Date de dépôt: 28.03.2000
(51) Int. Cl.: A61B 17/15

(54) **Guide de coupe d'une tibia comportant une poignée de réglage**
Tibiale Sägeführung mit Regelungshandgriff
Tibial cutting guide with adjustment handgrip

(30) Priorité: 01.04.1999 FR 9904089
(43) Date de publication de la demande: 04.10.2000
(73) Titulaire: Aesculap Société par actions simplifiée, 52000 Chaumont (FR)
(72) Inventeur: Millard, Thierry, 52340 Le Puits des Mezes (FR); Biegun, Jean-François, 52000 Chaumont (FR)
(74) Mandataire: Eidelsberg, Victor Albert

(56) Documents cités:
- FR-A- 2 720 629
- US-A- 5 451 228
- US-A- 5 704 941

## Description

La présente invention concerne un dispositif de mise en position d'un tibia par rapport à un guide de coupe, le guide de coupe pouvant alors guider une lame de scie pour la résection du tibia suivant un plan de résection donné, pour permettre la mise en place d'une prothèse du genou.

Les dispositifs de mise en position de ce genre sont déjà connus de l'art antérieur. Il comporte en général une potence, des moyens d'immobilisation distale du tibia destinés à immobiliser l'extrémité distale du tibia en une position donnée, ces moyens d'immobilisation distale étant montés coulissants sur la potence, avec possibilité de blocage, suivant au moins une direction, de préférence deux directions perpendiculaires, dans un plan perpendiculaire à la potence, des moyens d'immobilisation proximale du tibia, destinés à immobiliser l'extrémité proximale du tibia en une position donnée et étant montés coulissants le long de la potence et un guide de coupe monté sur la potence.

Ces dispositifs de l'art antérieur (par exemple de FR-A-2720629, sur lequel le préambule de la revendication 1 est basé, ou de US-A-5451228) présentent plusieurs inconvénients.

Le plan de résection choisi tient compte de l'inclinaison du tibia dans le plan antéro-postérieur ou sagittal, et de l'inclinaison du tibia dans le plan frontal (varus-valgus). Il est donc nécessaire, lorsque l'on positionne le guide de coupe par rapport au tibia immobilisé, de pouvoir régler l'angle formé entre la potence et le tibia dans le plan sagittal (pente postérieure) et de préférence l'angle formé entre la potence et le tibia dans le plan frontal (varus-valgus). Or, pour permettre ces réglages ainsi que celui de la hauteur du guide de coupe, ces dispositifs de l'art antérieur sont constitués d'un grand nombre de composants d'assemblage compliqués et difficiles à manipuler.

L'invention vise à pallier ces inconvénients, et notamment un dispositif de mise en position du genre décrit précédemment qui soit plus simple à prendre et à manipuler, notamment lors du réglage des inclinaisons du tibia par rapport à la potence dans les plans sagittal et de préférence frontal et de la hauteur du guide de coupe par rapport au tibia.

Suivant la présente invention, le dispositif de mise en position d'une extrémité proximale d'un tibia par rapport à un guide de coupe est caractérisé en ce qu'il comporte une poignée de pistolet ayant un chien et une détente et fixée à la potence et dont le chien agit sur des moyens qui permettent le réglage de la position relative des moyens d'immobilisation proximale par rapport à la potence avec possibilité de blocage et dont la détente agit sur des moyens qui permettent le réglage de l'angle (pente postérieure) entre la potence et le tibia dans le plan sagittal.

En prévoyant ainsi une poignée de pistolet à chien et détente, le dispositif permet au chirurgien une mise en position simple du tibia par rapport au guide de coupe. En effet, le positionnement des moyens d'immobilisation proximale s'effectue simplement en sollicitant le chien et en faisant coulisser ces moyens le long de la potence, puis, une fois la bonne position trouvée, en relâchant le chien, ce qui bloque en position les moyens d'immobilisation proximale, avec le plan de résection choisi en face du guide de coupe. Ensuite, on actionne la détente qui permet de régler la pente postérieure du tibia puis on relâche la détente pour bloquer la valeur réglée de la pente postérieure et ensuite à l'aide de la poignée, suivant un perfectionnement de l'invention, sans appuyer ni sur le chien, ni sur la détente, on règle par coulissement dans le plan frontal l'angle de varus-valgus.

Suivant un perfectionnement de l'invention, les moyens qui permettent un réglage de l'angle entre le tibia et la potence dans le plan sagittal sont constitués d'un levier dont une extrémité coopère avec une pièce de poussée dont l'extrémité inférieure comporte des dentelures qui engrènent avec des dentelures d'un bras de support, l'action de poussée sur la détente entraînant la sortie de l'engrènement des dentelures de la pièce avec les dentelures et le relâchement de la détente l'engrènement de ces mêmes dentelures de la seconde pièce de poussée avec les dentelures du bras, pour ainsi régler l'angle entre la potence et le tibia dans le plan sagittal. Le dispositif suivant ce perfectionnement est de fabrication simple.

Suivant un perfectionnement de l'invention, le bras support comporte une plaque coulissant dans un rail de guidage formé dans un support des moyens d'immobilisation distale du tibia, ce coulissement étant réalisé avec possibilité de blocage par coopération d'un ergot avec des évidements formés les uns à la suite des autres dans le rail de guidage, les évidements recevant successivement l'ergot pour un blocage de la plaque par rapport aux moyens d'immobilisation distale, pour ainsi régler l'angle entre le tibia et la potence dans le plan frontal (angle de varus/valgus).

Suivant un perfectionnement de l'invention, les moyens d'immobilisation proximale du tibia comportent une tige pouvant coulisser dans la potence, la tige comportant des cannelures, les cannelures engrenant avec des dentelures d'une première pièce de poussée dont le déplacement est actionné par le chien, la poussée du chien entraînant la sortie de prise des dentelures de la pièce avec les cannelures pour permettre un coulissement de la tige par rapport à la potence et le relâchement de l'appui sur le chien entraînant la mise en prise des dentelures de la première pièce de poussée avec les cannelures pour bloquer en position la tige par rapport à la potence, et donc les moyens d'immobilisation proximale par rapport au tibia.

Suivant un perfectionnement de l'invention, le nombre de cannelures sur le bras support correspond à un domaine de réglage déterminé à l'avance de l'angle de pente postérieure, par exemple entre 0° et 8°.

Suivant un perfectionnement de l'invention, le nombre d'évidements dans le rail de guidage correspond à un domaine de réglage déterminé à l'avance de l'angle de varus/valgus par exemple entre -3° et 3°.

Suivant un perfectionnement de l'invention, on prévoit que le guide de coupe peut coulisser, en étant bloqué en rotation, par rapport à la potence, dans le sens de la hauteur, indépendamment des moyens d'immobilisation proximale. Cela permet un réglage encore plus fin de la position du guide de coupe, et par conséquent de la position de la résection tibiale.

Aux dessins annexés, donnés uniquement à titre d'exemple, il est représenté un mode de réalisation de l'invention. Aux dessins :
la figure 1 est une vue de côté d'un dispositif suivant l'invention,

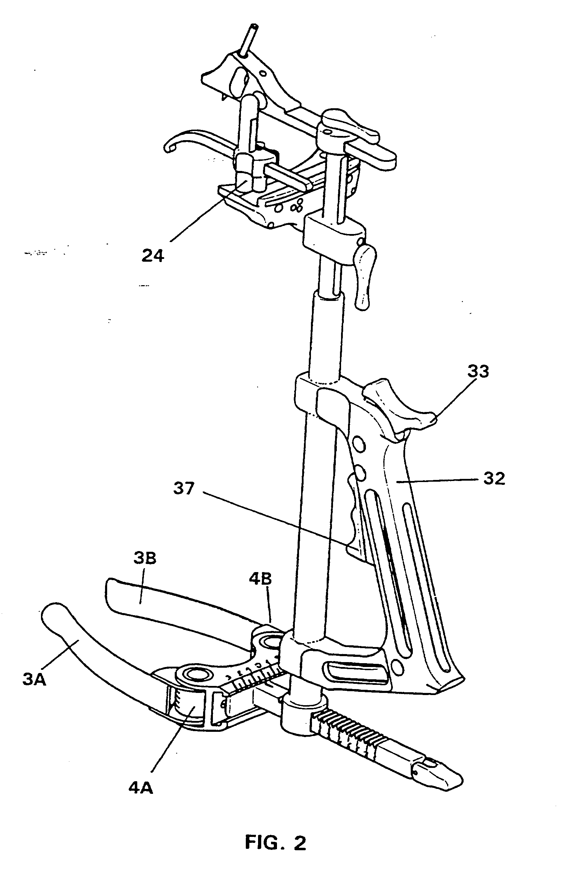

la figure 2 est une vue en perspective d'un dispositif suivant l'invention, et

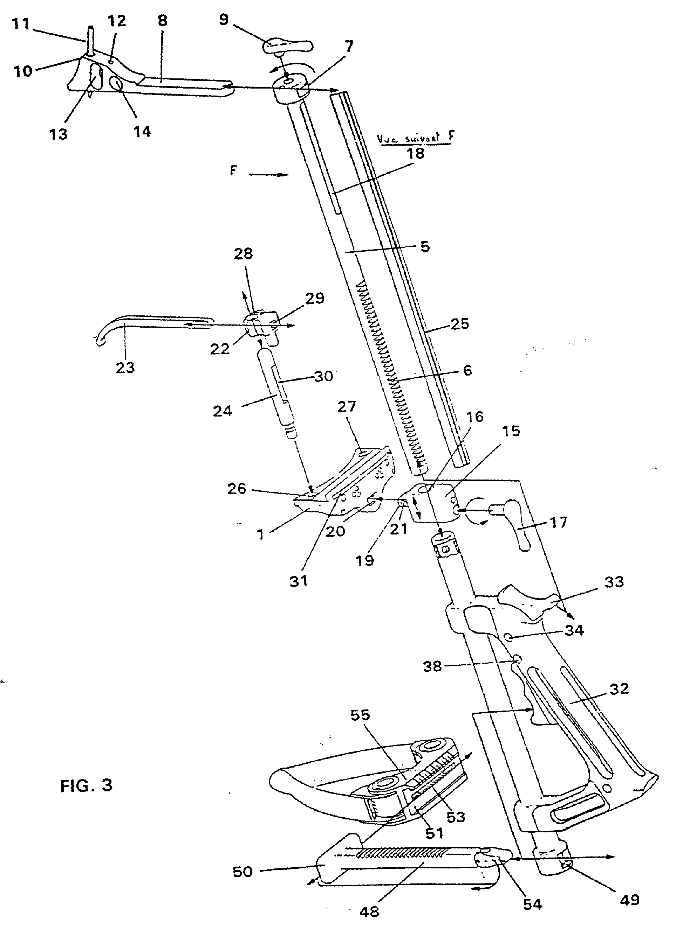

la figure 3 est une vue en perspective du dispositif de la figure 1, les différents éléments formant le dispositif étant à l'état non assemblé pour permettre une meilleure compréhension de la figure.

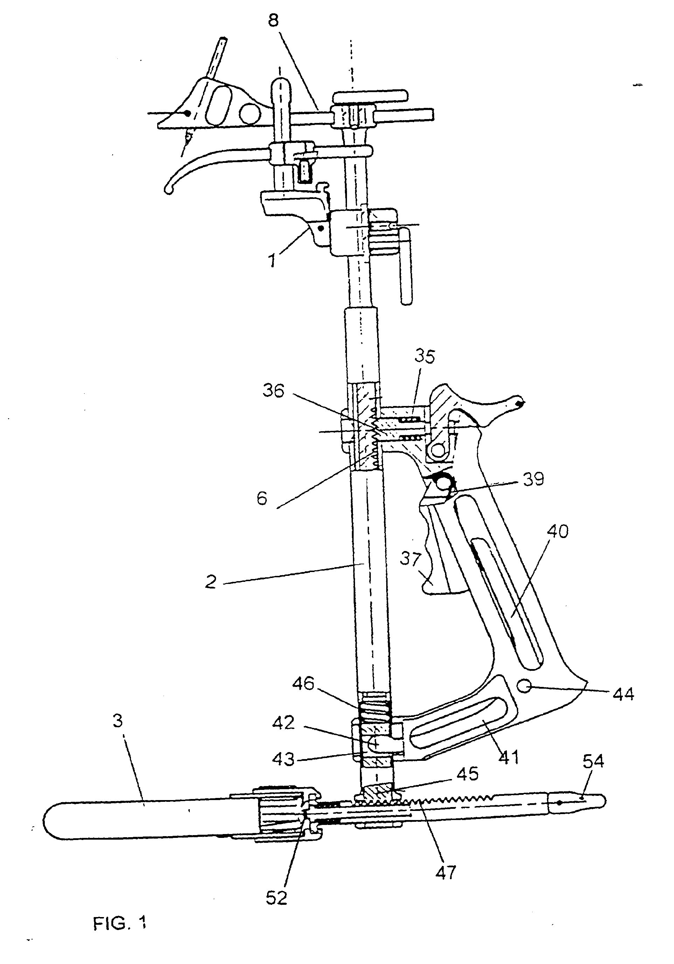

A la figure 1, le dispositif de mise en position d'une extrémité proximale d'un tibia (non représenté) par rapport à un guide de coupe 1 comporte une potence 2 cylindrique oblongue. Une pince 3 constituée de deux fourches 3A, 3B destinées à venir enserrer la malléole du tibia articulée à un support 55 et de deux ressorts 4A, 4B sollicitant les deux fourches de manière à serrer la malléole est montée à la potence 2 de manière à pouvoir coulisser perpendiculairement au plan comportant le tibia et la potence (le plan sagittal). La potence 2 oblongue creuse reçoit une tige 5 coulissante dans la partie supérieure de la potence 2. Cette tige 5 coulissante comprend à son extrémité inférieure des cannelures 6 qui, cela sera décrit plus tard, permettent le positionnement avec blocage de la tige 5 par rapport à la potence 2. La partie supérieure de la tige 5 comprend un alésage 7 de section transversale rectangulaire qui reçoit un doigt de fixation 8 coulissant dans cet alésage 7 et qui peut être bloqué par une vis 9 en une position choisie. Le doigt de fixation 8 comporte un alésage cylindrique 10 recevant un clou 11 imperdable destiné à être fiché, pour s'y fixer, à la partie proximale du tibia, notamment dans le massif des épines du tibia. Un autre alésage 12 est également prévu pour une autre broche ou clou. Deux évidements 13 et 14 de traversée sont formés dans le doigt 8, afin d'alléger le doigt et donc l'ensemble du dispositif. Un support 15 de guide de coupe peut coulisser sur la partie supérieure de la tige 5. Ce coulissement s'effectue de manière indépendante du coulissement de la tige 5 dans la potence 2. Le support 15 comprend un alésage 16 par lequel passe la tige 5 et par lequel le support 15 peut coulisser sur cette tige 5. Une vis 17 permet le blocage en position souhaitée du support 15 de guide de coupe sur la tige 5. La rotation du support 15 par rapport à l'axe longitudinal de la tige 5 est empêchée par le fait de prévoir au niveau de la tige 5 où peut coulisser le support 15 une rainure 25 longitudinale et un ergot dans le support 15, ce qui permet ainsi d'empêcher une rotation du support 15 par rapport à la tige 5. Le guide de coupe 1 est fixé au support 15 de manière simple, par exemple par la coopération d'une languette 19 avec un alésage 20 du guide de coupe de forme de section transversale complémentaire, avec encliquetage séparable, et à cet effet la languette 19 présente un orifice transversal 21 de réception d'une tige creuse contenant un ressort qui sollicite vers l'extérieur une bille de retenue destinée à venir se loger dans un cran correspondant ménagé dans la surface de l'alésage 20 du guide de coupe. Ainsi, aussi bien pour le montage du guide de coupe sur la languette que pour son démontage, il suffit de déplacer manuellement ces deux éléments l'un par rapport à l'autre en forçant la bille vers l'intérieur de l'orifice 21, cette bille sortant d'elle-même soit lorsque les deux éléments sont séparés, soit lorsque le guide de coupe est enfiché dans la languette 19. Le guide de coupe comporte dans sa partie supérieure un ensemble palpeur amovible comportant un corps 22, un doigt de palpage 23 et une colonne 24. La colonne 24 s'enfiche dans le guide 1 de coupe dans un alésage 26 gauche ou 27 droit. Un système d'encliquetage à bille peut être prévu. Le corps 22 coulisse sur la colonne 24 par l'intermédiaire d'un alésage 28, tandis que le doigt 23 de palpage coulisse dans un alésage 29 transversal à l'alésage 28.

Le coulissement du corps 22 sur la colonne 24 peut s'effectuer sans rotation mutuelle, la section transversale de l'alésage 28 étant certes circulaire, complémentaire de celle de la colonne 24, mais la coopération du doigt 23 avec l'ouverture 30 empêche une rotation du corps 22. De même, le coulissement du doigt 23 s'effectue sans rotation possible, la section transversale de l'alésage 29 étant carrée, comme celle du doigt 23. Il est prévu dans la colonne 24 une ouverture 30 permettant le passage à travers elle du doigt 23. Le blocage du doigt 23 et du corps 22 sur la colonne 24 est obtenu par pression d'une partie du corps 22 sur la colonne 24, grâce à un ressort positionné à l'intérieur du corps 22. Le doigt de palpage permet ainsi de matérialiser une hauteur de résection tibiale.

Le guide de coupe 1 comporte de manière classique un système de contreguidage de lame de scie ou une fente 31 pour le passage d'une lame de scie.

Il est fixé à la potence 2 une poignée 32 de revolver. Cette poignée 32 de revolver comprend dans sa partie supérieure un chien 33 articulé à un axe de rotation 34, le chien 33 coopérant avec un ressort 35 qui sollicite une première pièce 36 de poussée dont l'extrémité proximale au ressort 35 comporte des dents de crémaillère pouvant s'engager dans les cannelures 6 de la tige 5. La poignée 32 de revolver comprend également une détente 37 articulée par rapport à un axe de rotation 38. Un ressort 39 sollicite la détente 37 en exerçant un couple de rappel par rapport à l'axe 38. La détente 37 coopère avec un levier 40 disposé à l'intérieur de la poignée 32. Ce levier 40 est coudé en comportant deux branches, une première branche qui coopère avec la détente 37, tandis que sa deuxième branche 41 pénètre par son extrémité 42 dans un alésage transversal 43 de la potence 2, dans sa partie inférieure. Le levier 40 est monté fixe à l'intérieur de la poignée 32, en étant articulé par rapport à un axe 44 au niveau de la partie où se réunissent les deux branches du levier 40. A la partie inférieure de la potence 2, l'extrémité 42 du levier 40 coopère avec une deuxième pièce de poussée 45 et un ressort de rappel 46, disposés dans la potence 2, en pouvant coulisser à l'intérieur de celle-ci. L'extrémité inférieure de la deuxième pièce de poussée 45 comprend des dents de crémaillères qui s'engagent dans des cannelures 47 d'un bras support 48 qui est de forme oblongue, de section transversale carrée et qui coulisse dans un alésage 49 de la potence 2 dans sa partie inférieure. L'alésage 49 est de section transversale carrée, complémentaire de la section transversale du bras support 48. Le coulissement s'effectue dans le plan médio-latéral. Ce bras support 48 comporte à une de ses extrémités une plaque 50 de guidage qui peut coulisser dans un rail de guidage 51 formé dans le support des pinces 3A, 3B. Le coulissement de la plaque 50 de guide s'effectue dans le plan perpendiculaire à la potence 2, dans la direction perpendiculaire à la direction de coulissement du bras support 48 dans l'alésage 49. La face extérieure de la plaque 50 (éloignée de la potence 2) comporte un ergot 52 qui peut s'encliqueter dans une rangée d'évidements 53 formée dans la paroi du rail 51 de guidage sur laquelle est guidée la plaque 50. L'ergot 52 peut s'encliqueter dans chaque évidement 53 en y étant poussé par un ressort à l'intérieur du bras support 48. A chaque position de l'ergot 52 dans un évidement 53, il correspond donc une certaine position du support des pinces 3A et 3B et donc un certain angle relatif entre le tibia maintenu par les pinces et la potence, dans le plan frontal.

Le réglage de la position du tibia par rapport au guide de coupe s'effectue de la manière suivante : L'utilisateur saisit le système de positionnement par sa poignée 32 de revolver et enserre les malléoles du tibia par les pinces 3A, 3B qui s'adaptent de manière serrée par l'action des ressorts 4A, 4B de rappel. L'utilisateur appuie ensuite sur le chien 33, avec le pouce, la rotation dans le sens des aiguilles d'une montre du chien 33 entraînant le relâchement du ressort 35 qui entraîne avec lui la première pièce 36 de poussée, les dents de la pièce 36 sortant alors de leur engrènement avec les cannelures 6. Avec son autre main, l'utilisateur peut alors régler en hauteur la tige 5 en la faisant coulisser dans la potence 2. Une fois la bonne hauteur du doigt de fixation 8 réglée, l'utilisateur relâche le chien 33 qui, par l'intermédiaire du ressort 35 repousse la pièce 36 de poussée en prise par ses dentelures avec les cannelures 6 de la tige 5. La tige 5 est alors bloquée et ne peut plus se déplacer par coulissement dans la potence 2. Après avoir relâché le chien, l'utilisateur fixe alors la partie supérieure du tibia (massif des épines) par le clou 11 imperdable, au niveau du centre du tibia. Il appuie ensuite sur la détente 37. L'action de poussée sur cette détente 37 agit sur la première branche du levier 40 en liaison avec cette détente 37 et entraîne le soulèvement de la seconde branche 41 du levier 40, l'extrémité 42 de la seconde branche 41 du levier 40 entraînant alors vers le haut la seconde pièce 45 de poussée, dont la dentelure à l'extrémité inférieure sort de son engrènement avec les dentelures 47 du bras support 48. Le bras 48 support peut alors coulisser dans l'alésage 49, perpendiculairement à la potence 2 et lorsque le réglage de l'angle dans le plan sagittal entre le tibia maintenu par ses malléoles dans les fourches 3A, 3B et la potence 2 est effectuée à la valeur souhaitée, l'utilisateur relâche la détente 37, qui est ramenée en position libre par le ressort de rappel 39. L'extrémité 42 de la seconde branche 41 du levier 40 s'abaisse alors et les dentelures de la pièce 45 de poussée reviennent en prise dans les dentelures 47 du bras support 48. On a ainsi réglé la pente postérieure du tibia par rapport à la potence. L'angle peut être choisit entre 0° et 8°, en prévoyant un nombre de dents 47 suffisants pour cette plage de réglage. Ensuite, on effectue le réglage de l'angle varus/valgus (dans le plan frontal) en faisant coulisser la plaque 50 dans son rail 51, en déplaçant la potence 2 par la poignée 32 dans le plan perpendiculaire à la figure 1 (plan frontal). Une fois le bon angle choisi, on place l'ergot 52 dans l'évidement 53 dans lequel il se trouve alors, par l'intermédiaire d'un levier 54. On a alors réglé le varus-valgus. Le nombre d'évidements 53 est prévu pour permettre un réglage de par exemple ± 3° de l'angle varus-valgus (plan frontal). Le réglage de la hauteur, de la pente postérieure et du varus/valgus ayant été réalisé, on règle ensuite le doigt de palpage 23 en le faisant coulisser sur la colonne 24 pour définir la hauteur exacte du plan de résection. La résection peut alors être effectuée. On peut alors faire coulisser le support 15 du guide de coupe pour amener le doigt de palpage 23 en contact avec l'os pour martérialiser précisément le plan de résection et bloquer le support 15 en position par la vis 17.

Le chien et la détente sont tous les deux intégrés à la poignée. L'utilisateur peut appuyer sur le chien avec le pouce et sur la détente avec l'index facilement,

## Revendications

1. Dispositif de mise en position d'un tibia par rapport à un guide (1) de coupe, comportant :
- une potence (2) ;
- des moyens (3A, 3B, 4A, 4B, 55) d'immobilisation distale du tibia destinés à immobiliser l'extrémité distale du tibia en une position donnée et montés sur la potence, en pouvant coulisser avec possibilité de blocage, suivant au moins une direction dans un plan perpendiculaire à la potence (2) ;
- des moyens (8, 9, 10, 11, 12, 13, 14) d'immobilisation proximale du tibia montés coulissants le long de la potence (2) ;
- un guide de coupe (1) monté sur la potence (2) ;
- une poignée (32), comportant un chien et fixée à la potence (2) et dont le chien (33) agit sur des moyens (6, 35, 36) qui permettent le réglage de la position relative des moyens (8, 9, 10, 11, 12, 13, 14) d'immobilisation proximale par rapport à la potence (2) avec possibilité de blocage; et
- une détente (37) qui agit sur des moyens (40, 41, 42, 43, 44, 45, 46, 47) qui permettent le réglage de l'angle (pente postérieure) entre la potence (2) et le tibia dans le plan sagittal **caractérisé en ce que** la détente est intégrée à la poignée.

2. Dispositif de mise en position d'un tibia par rapport à un guide (1) de coupe suivant la revendication 1, **caractérisé en ce que** les moyens qui permettent un réglage de l'angle entre le tibia et la potence dans le plan sagittal sont constitués d'un levier (40) dont une extrémité (42) coopère avec une pièce (45) de poussée dont l'extrémité inférieure comporte des dentelures qui engrènent avec des dentelures (47) d'un bras (48) de support des moyens d'immobilisation distale du tibia, l'action de poussée sur la détente (37) entraînant la sortie de l'engrènement des dentelures de la pièce (45) avec les dentelures (47) et le relâchement de la détente (37) l'engrènement de ces mêmes dentelures de la seconde pièce (45) de poussée avec les dentelures (47) du bras (48), pour ainsi régler l'angle entre la potence (2) et le tibia dans le plan sagittal.

3. Dispositif de mise en position d'un tibia par rapport à un guide (1) de coupe suivant la revendication 2, **caractérisé en ce que** le bras support (48) comporte une plaque (50) coulissant dans un rail (51) de guidage formé dans un support (55) des moyens d'immobilisation distale du tibia, ce coulissement étant réalisé avec possibilité de blocage par coopération d'un ergot (52) avec des évidements (53) formés les uns à la suite des autres dans le rail (51) de guidage, les évidements recevant successivement l'ergot (52) pour un blocage de la plaque (50) par rapport aux moyens d'immobilisation distale, pour ainsi régler l'angle entre le tibia et la potence dans le plan frontal (angle de varus/valgus).

4. Dispositif de mise en position proximale d'un tibia par rapport à un guide (1) de coupe suivant la revendication 1 ou 2, **caractérisé en ce que** les moyens d'immobilisation proximale du tibia comportent une tige (5) pouvant coulisser dans la potence (2), la tige (5) comportant des cannelures (6), les cannelures (6) engrenant avec des dentelures d'une première pièce (35) de poussée dont le déplacement est actionné par le chien (33), la poussée du chien (33) entraînant la sortie de prise des dentelures de la pièce (35) avec les cannelures (6) pour permettre un coulissement de la tige (5) par rapport à la potence (2) et le relâchement de l'appui sur le chien (33) entraînant la mise en prise des dentelures de la première pièce (36) de poussée avec les cannelures (6) pour bloquer en position la tige (5) par rapport à la potence (2), et donc les moyens d'immobilisation proximale par rapport au tibia.

5. Dispositif de mise en position d'un tibia par rapport à un guide (1) de coupe suivant l'une des revendications précédentes, **caractérisé en ce que** le nombre de cannelures sur le bras support (48) correspond à une possibilité de réglage de l'angle de pente postérieure compris entre par exemple 0° et 8°.

6. Dispositif de mise en position proximale d'un tibia par rapport à un guide (1) de coupe suivant l'une des revendications 3 à 5, **caractérisé en ce que** le nombre d'évidements (53) dans le rail de guidage (51) correspond à un domaine de réglage possible de l'angle de varus/valgus compris entre par exemple -3° et 3°.

7. Dispositif de mise en position d'un tibia par rapport à un guide (1) de coupe suivant l'une des revendications précédentes, **caractérisé en ce que** le guide de coupe est monté coulissant et bloqué en rotation par rapport à la potence (2) dans le sens de la hauteur indépendamment des moyens (8, 9, 10, 11, 12, 13, 14) d'immobilisation proximale.

## Claims

1. A device for positioning a tibia against a cutting guide (1) including:
- a support bracket (2);
- means (3A, 3B, 4A, 4B, 55) of distally immobilising the tibia, which are designed to immobilise the distal extremity of the tibia in a given position and are mounted on the support bracket, being able to slide and be locked into position, following at least one direction perpendicular to the support bracket (2);
- means (8, 9, 10, 11, 12, 13, 14) of proximally immobilising the tibia, which are mounted on and can slide along the support bracket (2);
- a cutting guide (1) mounted on the support bracket (2);
a handle (32) incorporating a hammer and fixed to the support bracket (2), and where the hammer (33) works with the devices (6, 35, 36) that enable the relative position of devices (8, 9, 10, 11, 12, 13, 14) for proximal immobilisation to be adjusted with regard to the support bracket (2) with the possibility of locking those in place; and
a trigger (37), which works with devices (40, 41, 42, 43, 44, 45, 46, 47) that enable the angle (posterior slope) between the support bracket (2) and the tibia to be adjusted in the sagittal plane, whereby the trigger is built in to the handle.

2. A device for positioning a tibia against a cutting guide (1) according to claim 1, whereby the devices which enable the angle between the tibia and the support bracket to be adjusted in the sagittal plane consist of a lever (40), one end (42) of which works together with a pushing piece (45), the lower end of which has teeth that engage with the teeth (47) of a supporting arm (48); means of distally immobilising the tibia, whereby the action of pushing on the said trigger (37) leads to the teeth of the pushing piece (45) disengaging from the teeth (47) of the supporting arm, and the release of the trigger (37), to the engagement of these same teeth of the second pushing piece (45) with the teeth (47) of the arm (48), thus adjusting the angle between the support bracket (2) and the tibia in the sagittal plane.

3. A device for positioning a tibia against a cutting guide (1) according to claim 2, whereby the supporting arm (48) comprises a plate (50) sliding in a guide rail (51), formed by a support (55) of the distal immobilising devices of the tibia, the said sliding action is implemented with the possibility of locking by co-operation between a pin (52) and a row of notches (53) formed on the guide rail (51), as the notches take the pin (52) one after the other to lock the plate (50) with regard to the distal immobilising devices, in order to adjust the angle between the tibia and the support bracket in the frontal plane (angle of varus/valgus).

4. A device for positioning a tibia against a cutting guide (1) according to claim 1 or claim 2, whereby the proximal immobilising devices for the tibia include a shaft (5) that can slide into the support bracket (2) the said shaft (5) including notches (6), which engage with the teeth of a first pushing piece (35), the movement of which is initiated by the hammer (33), the pushing of said hammer (33) leading to the release of the teeth of the pushing piece (35) from the notches (6) to allow the shaft (5) to slide in the support bracket, and releasing the pressure on the hammer (33) causes the teeth of the first pushing piece (36) to engage with the notches (6) to lock the shaft (5) into position with regard to the support bracket (2), therefore locking the proximal immobilising devices with regard to the tibia.

5. A device for positioning a tibia against a cutting guide (1) according to one of the previous claims, whereby the number of notches on the supporting arm (48) correspond to a possibility of adjusting the angle of the posterior slope, from between 0° and 8° degrees, for example.

6. A device for positioning a tibia against a cutting guide (1) according to one of claims 3-5, whereby the number of notches (53) on the guide rail (51) corresponds to a possible range of adjustment of the angle of varus/valgus, of between -3° and 3° for example.

7. A device for positioning a tibia against a cutting guide (1) according to one of the previous claims, whereby the cutting guide is mounted so as to slide but not rotate with regard to the support bracket (2) in a vertical direction, independently of the proximal immobilising devices (8, 9, 10, 11, 12, 13, 14).

## Patentansprüche

1. Vorrichtung zur Positionierung eines Schienbeins relativ zu einer Führung (1) für einen Schnitt, umfassend:
- einen Trägerarm (2);
- Mittel (3A, 3A, 4A, 4B, 55) zur distalen Festsetzung des Schienbeins zum Festsetzen des distalen Endes des Schienbeins in einer vorbestimmten Position, wobei die Mittel (3A, 3A, 4A, 4B, 55) verschiebbar in einer Ebene senkrecht zu dem Trägerarm (2) und blockierbar an dem Trägerarm (2) befestigt sind;
- Mittel (8, 9, 10, 11, 12, 13, 14) zur proximalen Festsetzung des Schienbeins, wobei die Mittel (8, 9, 10, 11, 12, 13, 14) längs zum Trägerarm (2) verschiebbar sind;
- eine Führung (1) für einen Schnitt, die an dem Trägerarm (2) befestigt ist;
- einen Handgriff (32), der an dem Trägerarm (2) befestigt ist, mit einem Sperrhebel (33), der auf Mittel (6, 35, 36) wirkt, die eine Einstellung der Position relativ zu den Mitteln (8, 9, 10, 11, 12, 13, 14) zur proximalen Festsetzung in Bezug auf den Trägerarm (2) mit Möglichkeit zum Blockieren erlauben; und
- einen Drücker (37), der auf Mittel (40, 41, 42, 43, 44, 45, 46, 47) wirkt, die die Einstellung eines Winkels (hintere Neigung) zwischen dem Trägerarm (2) und dem Schienbein in der sagittalen Ebene erlauben,
**dadurch gekennzeichnet, dass** der Drücker (37) an dem Handgriff (32) angeordnet ist.

2. Vorrichtung zur Positionierung eines Schienbeins relativ zu einer Führung (1) für einen Schnitt nach Anspruch 1, **dadurch gekennzeichnet, dass** die Mittel, die eine Einstellung des Winkels zwischen dem Schienbein und dem Trägerarm (2) in der sagittalen Ebene erlauben, einen Hebel (40) umfassen, von dem ein Ende (42) mit einem Druckstück (42) zusammenwirkt, das an seinem äußeren Ende Zähne aufweist, die mit Zähnen (47) eines Tragarms (48) der Mittel (3A, 3A, 4A, 4B, 55) zur distalen Festsetzung des Schienbeins in Eingriff sind, wobei ein Drücken des Drückers (37) ein Lösen des Eingriffs des Druckstücks (45) aus den Zähnen (47) und das Loslassen des Drückers (37) das Eingreifen der genannten Zähne des zweiten Druckstücks (42) in die Zähne des Tragarms (48) bewirkt, um so den Winkel zwischen dem Trägerarm (2) und dem Schienbein in der sagittalen Ebene einzustellen.

3. Vorrichtung zur Positionierung eines Schienbeins relativ zu einer Führung (1) für einen Schnitt nach Anspruch 2, **dadurch gekennzeichnet, dass** der Tragarm (48) eine Platte (50) umfasst, die in einer Führungsschiene (51), die in einem Träger (55) der Mittel zur distalen Festsetzung des Schienbeins ausgebildet ist, gleitend gelagert ist, wobei die Gleitlagerung mit Mitteln zum Blockieren durch Zusammenwirken eines Nockens (52) mit Aussparungen (53) ausgestattet ist, die eine neben der anderen in der Führungsschiene (51) ausgebildet sind, wobei die Aussparungen aufeinander folgend den Nocken aufnehmen, um die Platte (50) in Bezug zu den Mitteln zum distalen Festsetzung zu blockieren, um so den Winkel zwischen dem Schienbein und dem Trägerarm in einer frontalen Ebene (Winkel Varus/Valgus) einzustellen.

4. Vorrichtung zur Positionierung eines Schienbeins relativ zu einer Führung (1) für einen Schnitt nach einem der Ansprüche 1 oder 2, **dadurch gekennzeichnet, dass** die Mittel zur proximalen Festsetzung des Schienbeins einen Schaft (5) umfassen, der gleitend in dem Trägerarm (2) gelagert ist, wobei an dem Schaft (5) Nuten (6) ausgebildet sind, die mit Zähnen eines ersten Druckstücks (35) zusammenwirken, das durch den Sperrhebel (33) verschiebbar ist, wobei das Betätigen des Sperrhebels (33) das Ausrücken der Zähne des ersten Druckstücks (35) aus den Nuten (6) bewirkt, um ein Verschieben des Schafts (5) in dem Trägerarm (2) zu erlauben, und das Loslassen des Sperrhebels (33) das Einrasten der Zähne des ersten Druckstücks (36) in die Nuten (6) bewirkt, um die Lage des Schafts (5) in dem Trägerarm (2) und damit die Mittel zur proximalen Festsetzung relativ zum Schienbein zu blockieren.

5. Vorrichtung zur Positionierung eines Schienbeins relativ zu einer Führung (1) für einen Schnitt nach einem der vorigen Ansprüche, **dadurch gekennzeichnet, dass** die Anzahl der Zähne auf dem Tragarm (48) einer Einstellmöglichkeit des hinteren Neigungswinkels von zum Beispiel 0° bis 8° entspricht.

6. Vorrichtung zur Positionierung eines Schienbeins relativ zu einer Führung (1) für einen Schnitt nach einem der Ansprüche 3 bis 5, **dadurch gekennzeichnet, dass** die Anzahl der Aussparungen (53) in der Führungsschiene (51) einer Einstellmöglichkeit des VarusNalgus- Winkels von zum Beispiel -3° bis 3° entspricht.

7. Vorrichtung zur Positionierung eines Schienbeins relativ zu einer Führung (1) für einen Schnitt nach einem der vorigen Ansprüche, **dadurch gekennzeichnet, dass** die Führung für einen Schnitt (1) relativ zum Trägerarm (2) längsverschiebbar und drehfest unabhängig von den Mitteln (8, 9, 10, 11, 12, 13, 14) zur proximalen Festsetzung gelagert ist.
